(19) Europäisches Patentamt
European Patent Office
Office européen des brevets

(11) Veröffentlichungsnummer: **0 248 369 A2**

(12) # EUROPÄISCHE PATENTANMELDUNG

(21) Anmeldenummer: 87107859.8

(22) Anmeldetag: 30.05.87

(51) Int.Cl.³: C 12 P 7/54

(30) Priorität: 02.06.86 DE 3618076

(43) Veröffentlichungstag der Anmeldung: 09.12.87 Patentblatt 87/50

(84) Benannte Vertragsstaaten: AT BE CH DE FR GB IT LI LU NL SE

(71) Anmelder: Kernforschungsanlage Jülich Gesellschaft mit beschränkter Haftung
Postfach 1913
D-5170 Jülich 1(DE)

(71) Anmelder: PFEIFER & LANGEN
Linnicher Strasse 48
D-5000 Köln 41(DE)

(72) Erfinder: Klemps, Robert
Gutenbergstrasse 11
D-5170 Jülich(DE)

(72) Erfinder: Schoberth, Siegfried, Dr.
Gutenbergstrasse 22
D-5170 Jülich(DE)

(72) Erfinder: Sahm, Hermann, Prof.
Wendelinusstrasse 71
D-5170 Jülich(DE)

(72) Erfinder: Swyzen, Werner
Grüner Weg 2
D-5177 Titz-Ameln(DE)

(54) **Verfahren zur mikrobiellen anaeroben Gewinnung von Essigsäure.**

(57) Die anaerobe Gewinnung von Essigsäure durch Vergärung mit Hilfe von homoacetogenen Bakterien in einer wäßrigen Lösung von vergärbaren Substanzen, insb. von Zucker, führt zu erhöhten Essigsäurekonzentrationen (über 3 %), wenn bei der Vergärung eine Substratlösung vorgelegt wird, die bereits (aus einer vorangehenden Produktion) verdünnte Essigsäure enthält. Für Fed-Batch-Betrieb werden etwa 0,5 - 2 %ige insb. ca 1 %ige Essigsäure vorgegeben. Die Fermentation erfolgt insb. zwischen 30 und 75° C bei pH 5 bis 7,5, eingestellt insb. mit etwa 10 M Natronlauge, mit Hilfe von Acetogenium kivui. Eine Aufkonzentrierung oder Immobilisierung von Biomasse ist zweckmäßig. Eine kontinuierliche Fermentation wird insb. in zwei hintereinandergeschalteten Fermentern mit etwa 1d und zumindest 2d Verweilzeit durchgeführt.

EP 0 248 369 A2

Croydon Printing Company Ltd.

Kernforschungsanlage Jülich GmbH    Pfeifer & Langen

B e s c h r e i b u n g

Verfahren zur mikrobiellen anaeroben Gewinnung von Essigsäure

Die Erfindung bezieht sich auf ein Verfahren zur anaeroben Gewinnung von Essigsäure, ausgehend von Substraten für homoacetogene Bakterien, insbesondere von Zuckerlösungen durch Gärung mit Hilfe von homoacetogenen Bakterien in wäßrigem Milieu.

Essigsäure ist ein für die chemische Industrie wichtiger und in zunehmendem Maße benötigter Grundstoff, der z. Zt. in großen Mengen ausgehend von Erdöl und Erdgas hergestellt wird. Ferner wird Essigsäure durch mikrobielle Umsetzung von Zucker durch alkoholische Gärung (1) und nachfolgende unvollständige Oxidation des Ethanols durch aerobe Essigsäurebakterien (2), wie im folgenden Schema kurz skizziert, hergestellt:

$$C_6H_{12}O_6 \xrightarrow{(1)} 2CH_3CH_2OH + 2CO_2$$

$$2CH_3CH_2OH + 2O_2 \xrightarrow{(2)} 2CH_3COOH + 2H_2O$$

Bei dieser zweistufigen Umwandlung von Glucose zu Essigsäure werden maximal 0,67 kg Essigsäure pro 1 kg Glucose gebildet, d.h. die Ausbeute kann bezogen auf die umgesetzte Glucose maximal nur 67 % betragen.

PT 1.814
nö/Fr

Mit Hilfe von anaeroben homoacetogenen Bakterien ist es dagegen möglich, Zucker praktisch quantitativ zu Essigsäure umzuwandeln:

$$C_6H_{12}O_6 \longrightarrow 3CH_3COOH \; .$$

Dieser Vorgang wird Homoacetatgärung genannt. Streng anaerob lebende Bakterien, die zu einer solchen Umwandlung von Glucose, Fructose oder Xylose in Essigsäure befähigt sind, sind seit langer Zeit bekannt, wie z.B. einige Stämme der Gattung Clostridium und seit kurzem Stämme von Acetobacterium und Acetogenium, die außer Zucker auch andere niedermolekulare Verbindungen wie Lactat, Pyruvat, Kohlenmonoxid, Formiat, Kohlendioxid und Wasserstoff, und Methanol zu Essigsäure umsetzen können.

Die einstufige Prozeßführung, quantitative Umsetzung von Zuckern bzw. Substraten zu Essigsäure und der Wegfall des bei aeroben Verfahren notwendigen Sauerstoffeintrags in die Bakteriensuspension lassen die Homoacetatgärung besonders günstig erscheinen. Ein erheblicher Nachteil der Homoacetatgärung besteht allerdings in der Essigsäureempfindlichkeit der Bakterien, die zu einer starken Hemmung der Essigsäurebildung mit zunehmender Konzentration führt, so daß normalerweise Essigsäuregehalte über etwa 3,5 % nicht erreicht werden können.

So geringe Konzentrationen an Essigsäure stehen einer wirtschaftlichen Anwendung des Verfahrens im Wege, und es ist daher Aufgabe der Erfindung, ein Verfahren zur Gewinnung von Essigsäure vorzusehen, mit dem höhere Gehalte von insbesondere 4 - 5 % durch Homoacetatgärung erzielt werden können.

Diese Aufgabe wird durch das erfindungsgemäße Verfahren gelöst, das dadurch gekennzeichnet ist, daß man die Fermentation in einem Fermenterverbund von zumindest zwei Fermentern durchführt.

Der Fermenterverbund kann in unterschiedlicher Weise realisiert werden und zwar,

a) mit einem ersten Fermenter, aus dem wiederkehrend ein Fermentervolumenanteil zwischen 20 und 80 % entnommen und in einen zweiten Fermenter überführt wird, in dem dieser Teil ausgegoren wird, während der erste Fermenter mit frischer Substratlösung bzw. Nährlösung aufgefüllt wird. Bei einer Fermentation mit Hilfe von Acetogenium kivui erfolgt dieser Austausch insbesondere nach 35 bis 55 Stunden.

b) gemäß einer demgegenüber bevorzugten Alternative werden zwei Fermenter im Wechselbetrieb verwendet, wobei jeweils ein solcher Teil des einen Fermenters vor Erreichen der Essigsäureendkonzentration in diesem Fermenter als Starterkultur und Essigsäurevorgabe für den anderen Fermenter verwendet wird, daß dessen Essigsäure bzw. Acetatstartkonzentration zwischen 0,5 und 2 % insb. um ca. 1 % liegt.

c) bei kontinuierlicher Arbeitsweise wird in zumindest zwei hintereinander geschalteten Fermentern gearbeitet, von denen der erste zufließende substrathaltige Nährlösung erhält, während der zweite mit dem Ablauf des ersten gespeist wird und die Fermenterkapazitäten derart aufeinander abgestimmt sind, daß die Verweilzeit im zweiten Fermenter zumindest gleich derjenigen der ersten, vorzugsweise aber zumindest verdoppelt und insbesondere verdreifacht ist. Die Verweilzeit im ersten Fermenter wird dabei so gewählt, daß im ersten Fermenter eine möglichst hohe Essigsäure- bzw. Acetatkonzentration erreicht wird, die Aktivität der mit dem Ablauf des ersten Fermenters in den zweiten gelangenden Biomasse jedoch noch für eine Nachgärung in diesem bis zu den gewünschten hohen Essigsäure- bzw. Acetatkonzentrationen ausreicht. Vorzugsweise wird im ersten Fermenter bis zu einer Essigsäurekonzentration von etwa 3 % gearbeitet.

Bei der vorstehend unter a) bis c) beschriebenen Verfahrensweise ist es möglich, Essigsäure bzw. Acetatkonzentrationen über 4 % zu erreichen, während durch einfachen batch-Betrieb oder durch eine kontinuierliche Fermentation in einem Fermenter lediglich Konzentrationen bis zu etwa 3,7 % erzielt werden.

Für die Fermentation können dabei homoacetogene Bakterien, wie bereits oben genannt, angewandt werden. Weitere Beispiele finden sich z. B. bei G. Gottschalk "Microbial Metabolism" 1985, 2. Auflage, Springer, New York, Seite 250. Als besonders geeignet hat sich jedoch Acetogenium kivui erwiesen, das aus diesem Grunde vornehmlich für die weiter unten mitgeteilten Untersuchungen verwendet wurde.

Die überraschend hohe Produktbildungsrate und das besonders rasche Wachstum von Acetogenium kivui wird durch Vergleich der Daten der nachfolgenden Tabelle ersichtlich, die Werte für die Essigsäurebildung mit Hilfe von Acetogenium kivui (ATCC 33488, DSM 2030) sowie zwei anderen homoacetogenen Bakterien, und zwar Acetobacterium woodii (DSM 1030, ATCC 29683) und Clostridium thermoaceticum (DSM 521) zeigt.

Tabelle 1: Bildung von Essigsäure aus Glucose durch A.woodii (1), Cl. thermoaceticum (2) und A. kivui (3)

| Stamm | Temp. Opt. (°C) | Prod.-Konz. (mM) | Prod.-Bildungs-rate (mmol/l.h) | Prod.-Ausbeute (%umges.Gluc.) | Zell-Aktiv. (nmol Acetat / min.mg Prot.) |
|---|---|---|---|---|---|
| 1 | 33 | 133 | 0,7 - 1,3 | um 80 | 20 |
| 2 | 57 | 610 | 7 - 12 | über 90 | 120 |
| 3 | 66 | 640 | 25 - 28 | 85 - 90 | 800 |

A. kivui ist ein nichtsporenbildendes, streng anaerobes, gram-negatives stäbchenförmiges Bakterium, das bei pH 6,4 und 66 °C optimal wächst.

Mutanten mit erhöhter Produktivität lassen sich in bekannter Weise aus den genannten Stämmen mit mutagenen Mitteln gewinnen. Man kann auch nach bekannten gentechnischen Methoden unter Erhöhung der Genzahl oder der Effektivität des Promotorsystems Systeme mit höheren Produktionsraten erzielen.

Bei Verwendung von Acetogenium kivui sollte die Nährlösung im Vergleich zu den bislang für Essigsäureherstellung angegebenen Werten einen relativ hohen ( etwa 2- bis 5-fachen) Spurenelementgehalt und einen relativ niedrigen (etwa 1/5 bis 1/10) Phosphatgehalt aufweisen.

Beim Wechselbetrieb mit zwei Fermentern (A und B) gemäß (b) wird mit Acetogenium kivui zweckmäßigerweise jeweils aus einem Fermenter (A) ca. 36 - 42 Stunden nach Füllung bzw. Inbetriebnahme etwa 25 % seines Inhalts in den anderen Fermenter (B) überführt, der zu 75 % mit Nährlösung gefüllt ist, wonach der eine Fermenter (A) nach Erreichen der Endkonzentration (ca. 50 - 60 Stunden nach Füllung bzw. Inbetriebnahme) geleert und erneut zu 75 % mit frischer Nährlösung gefüllt wird.

Bei der Arbeitsweise gemäß (a) und (b) kann die Gesamtmenge der zu vergärenden Glucose bereits in der zugegebenen Nährlösung sein oder aber man kann mit einem verringerten Anfangsgehalt in der Nährlösung vorzugsweise unter 4 % und insb. um etwa 2 % (± 0,4 %) arbeiten und schubweise für eine Glucosenachlieferung bis zur Zugabe der gewünschten Gesamtmenge sorgen.

Bei kontinuierlicher Fermentation in zumindest zwei hintereinandergeschalteten Fermentern (jeweils mit pH-Kontrolle, z. B. durch NaOH (10 M) Zugabe) wurden bei Verwendung von Acetogenium kivui gute Ergebnisse mit Verweilzeiten von 18 bis 24 Stunden im ersten Fermenter und einem Volumenverhältnis von 1 : 3 zwischen erstem und zweitem Fermenter erhalten.

Für die Fermentation können beliebige von homoacetogenen Bakterien verwertbare Substrate und insb. Zucker angewandt werden, bei Temperaturen im Bereich von 30 bis 75 °C und pH-Werten im Bereich von 5 bis 7,5. Die Fermentation kann mit wachsenden und mit nichtwachsenden (ruhenden) Zellen durchgeführt werden. Zweckmäßig ist eine Immobilisierung von Biomasse im Fermenter oder deren Abtrennung vom Ablauf und Rückführung, da durch aufkonzentrierte Biomasse eine Verkürzung der Fermentationsdauer (z. B. um 1/3 durch 10-fache Zellkonzentration) erreicht werden kann.

Für die erste Inbetriebnahme ist insb. bei Arbeitsweise gemäß (a) und (b) die Anzüchtung von Inoculum zweckmäßig, wozu insbesodere mit 0,5 bis 2 % Acetat enthaltender Nährlösung ggfs. mit schubweiser Substratnachlieferung bis zur Erzielung einer Gesamtessigsäure- bzw. Acetatkonzentration von mehr als 4 % fermentiert wird.

Bei Verwendung von Acetogenium kivui wird dabei mit einem pH-Wert von 6,2 bis 6,8, einer Temperatur von 60 - 70° C gearbeitet und zunächst eine Fermentation von glucosehaltiger 0,5 bis 2 %iger Acetatlösung durchgeführt, deren Glucosekonzentration durch wiederkehrende Nachlieferung von Glucose unter 4 % insb. um 2 % bis zu einer Gesamtzugabe von 40 g/l Glucose gehalten wird bis eine Gesamtkonzentration von mehr als 4 % Essigsäure bzw. Acetat erreicht ist.

Dabei wird vorzugsweise mit ca. 170 mM Acetatlösung, insb. Natriumacetatlösung, bei pH 6,4 und 66° C gearbeitet. Zur Neutralisation dient dabei insbesondere etwa 10 M Natronlauge, aber selbstverständlich können andere und anders konzentrierte Neutralisierungsmittel vorgesehen werden.

Weitere Besonderheiten ergeben sich aus den nachfolgenden Beispielen.

Beispiel 1:

1.1 Vorkulturen: Die Nährlösung unterschied sich von der weiter unten (1.2) beschriebenen wie folgt (Angaben in g/l): $K_2HPO_4$, 7; $KH_2PO_4$, 5,5; Hefeextrakt, 2; $(NH_4)_2SO_4$, 0,5; NaCl, 1; KCl, 0,5; Typton fehlte; zusätzlich $NaHCO_3$, 10; die Glucosekonzentration betrug 2 % (w/v). Die Nährlösung wurde randvoll in 100 ml-Schraubdeckelfläschchen gefüllt. Der Inhalt einer mit gefriergetrocknetem Acetogenium kiviu gefüllten Ampulle wurde mit 2 ml dieser Nährlösung aufgeschwemmt, in eine Schraubdeckelflasche mit Nährlösung überführt und bei 66° C inkubiert. In der Regel lag nach 34 bis 36 h die Trübung ($OD_{660}$) der Kultur über 2. Hierauf wurden aus dieser aktiven Kultur 10 % entnommen und in neue Nährlösung überimpft. Nach ca. 10 h Inkubation bei 66° C lag die Trübung wieder über 2 und die Kultur konnte zum Beimpfen der Fermenter (s.u., 1.2) verwendet werden.

1.2: In einen 2l-Fermenter wurde eine Nährlösung folgender Zusammensetzung gegeben:

| | | |
|---|---|---|
| $H_2O$ bidest | | 1,0 l |
| Hefeextrakt | (Merck) | 5,0 g |
| Trypton | (BBL 11921) | 3,0 g |
| $(NH_4)_2SO_4$ | | 2,0 g |
| $MgSO_4 x 2H_2O$ | | 0,2 g |
| $CaCl_2 x 2H_2O$ | | 0,15 g |
| KCl | | 0,5 g |
| NaCl | | 1,0 g |
| $KH_2PO_4$ | | 1,5 g |
| $K_2HPO_4$ | | 1,5 g |
| $NiCl_2$ | 1 mM | 1,3 ml |
| SeWo-Lösung | s. Rezept | 0,6 ml |
| $Co(NO_3)_2 x 6H_2O$ | 0,1 M | 1,1 ml |
| $(NH_4)_2Fe(SO_4)_2$ | 0,1 M | 1,2 ml |
| $Na_2MoO_4 x 2H_2O$ | 0,1 M | 1,1 ml |
| SL 9 | s. Rezept | 1,2 ml |
| $Na_2S$ | 1 M | 1,0 ml |

Glucose je nach gewünschter Konzentration, als konzentrierte Lösung zugeben

| | |
|---|---|
| pH | 6,4 |
| Temperatur | 66 - 68 °C |

<u>SeWo-Lösung</u>

| | |
|---|---|
| $Na_2SeO_3 x 5H_2O$ | 3 mg/l |
| $Na_2WoO_4 x 2H_2O$ | 4 mg/l |

<u>SL 9</u>

| | |
|---|---|
| $H_2O$ bidest | 1000 ml |
| Nitrilotriessigsäure (NTE) | 12,5 g |
| $FeCl_2 x 4H_2O$ | 2,0 g |
| $ZnCl_2$ | 70 mg |
| $MnCl_2 x 4H_2O$ | 100 mg |
| $H_3BO_3$ | 6 mg |
| $CoCl_2 x 6H_2O$ | 190 mg |
| $CuCl_2 x 2H_2O$ | 2 mg |
| $NiCl_2 x 6H_2O$ | 24 mg |
| $Na_2MoO_4 x 2H_2O$ | 36 mg |

Dazu wurden ca. 170 m mol/l Natriumacetat hinzugefügt sowie 2 % Glucose und die Fermentationsbrühe mit Zellen von Acetogenium kivui aus einer Vorkultur beimpft (10 % v/v Inokulum). Nach 20 Stunden wurde nochmals etwa 1,7 % Glucose zum Fermenter hinzugegeben.

Nach insgesamt etwa 40 bis 50 Stunden lag die Gesamtkonzentration an Essigsäure im Fermenter bei etwa 4,3 bis 4,6 %.

Aus diesem Fermenter wurde dann soviel Bakteriensuspension in einen neuen mit frischer Nährlösung gefüllten Fermenter steril überführt, daß die Essigsäurekonzentration in diesem Fermenter bei etwa 0,8 bis 1 % lag. Nach ca. 50 Stunden waren wiederum etwa 3,4 bis 3,6 % Essigsäure gebildet, unter Erzielung einer Gesamtkonzentration von 3,9 bis 4,4 % Essigsäure (siehe Tabelle 2).

Dieser Vorgang ließ sich jeweils ohne Aktivitätsverlust der Bakterien wiederholen. Dabei wurden nach jedem Zyklus etwa 75 % des Fermentervolumens als 4 bis 4,5%ige Essigsäurelösung in einer Zeit von ca. 50 Stunden gewonnen.

| Versuch Nr. | vorgelegte Menge Na-Acetat (mM) | Gesamtmenge an Essigsäure (mM) | / % | netto Menge (mM) | Zeit (h) | maximale $OD_{660}$ | Überimpfung nach (h) |
|---|---|---|---|---|---|---|---|
| 1 | 158 | 744 | 4,4 | 586 | 48,0 | 8,5 | 31 |
| 2 | 80 | 644 | 3,9 | 564 | 41,5 | 8,9 | 41,5 |
| 3 | 100 | 669 | 4,0 | 569 | 49,5 | 9,1 | 49,5 |
| 4 | 78 | 693 | 4,2 | 615 | 46,5 | 9,0 | 46,5 |
| 5 | 130 | 730 | 4,3 | 600 | 57,0 | 8,9 | 50,0 |
| 6 | 170 | 690 | 4,1 | 520 | 49,5 | 8,3 | abgebrochen |

Tabelle 2: Ergebnisse der Fermentationsversuche mit A.kivui. In Versuch Nr. 1 wurde 158 mM Na-Acetat vorgelegt. Die Fermenterzellen wurden dann nach 31 Stunden auf einen neuen Fermenter überimpft; dasselbe geschah mit allen folgenden aufgeführten Fermenterläufen.

Die Isolierung und Reinigung der Essigsäure aus dem Kulturmedium wird durch konventionelle Methoden vorgenommen.

Beim beschriebenen Wechselbetrieb mit zwei parallel geschalteten Fermentern kann das Verhältnis von Nährlösung zu essigsäurehaltiger Fermentationsbrühe selbstverständlich von 3 : 1 verschieden sein und je nach Essigsäurekonzentration der Fermentationsbrühe bis zu etwa 9 : 1 reichen, vorausgesetzt, daß eine Anfangskonzentration von Essigsäure (bzw. Acetat) im Ansatz von zumindest 0,5 % erreicht wird.

Beispiel 2

Das Verfahren konnte ohne Schwierigkeiten im 10 l-Maßstab mit zumindest gleich gutem Erfolg durchgeführt werden.

Die nachfolgende Tabelle zeigt die im 10 l-Maßstab unter jeweiliger Vorlage von ca. 1%iger Essigsäure mit Acetogenium Kivui bei ca. 66 - 68° C unter Überimpfung von jeweils 20 - 25 % Fermenterinhalt als Inoculum für den jeweils nächsten Fermentergang erhalten würden

| Fermenterlauf Nr. | Essigsäureendkonz. (%) | nach Std. | Zeit bis Überimpfung (Std.) |
|---|---|---|---|
| 1 | 4,8 | 64 | 41 |
| 2 | 4,2 | 70,5 | 36 |
| 3 | 4,6 | 60 | 44 |
| 4 | 3,9 | 68 | 43,5 |
| 5 | 4,2 | 48 | Ende |

Beispiel 3

Die Ergebnisse einer kontinuierlichen Produktion mit zwei hintereinandergeschalteten Fermentern von 1 l u. 3 l Kapazität sind in der nachfolgenden Tabelle zusammengefaßt:

| | Fermenter 1 | Fermenter 2 |
|---|---|---|
| Verweilzeit (Stunden) | 21 | 63 |
| Trübung (OD 660 nm) | 9,5 | 8,2 |
| Essigsäure (%) | 3 | 4,1 |
| (mM) | 500 | 679 |
| Restglucose (%) | 1,24 | 0,04 |
| (mM) | 69,12 | 2,16 |
| Bakterielles Protein (mg/l) | 1113 | 978 |

Die vorstehend an Hand von Umsetzungen mit Acetogenium kivui beschriebene Verfahrensweise ist selbstverständlich auch mit anderen homoacetogenen Bakterien anwendbar, wobei durch die erfindungsgemäße Prozeßsteuerung jeweils eine Verbesserung gegenüber der bislang bekannten Arbeitsweise erwartet werden kann.

Ferner könnte natürlich der pH-Bereich für die Fermentation mit neu isolierten bzw. gentechnologisch hergestellten Bakterien am unteren Ende unter pH 5, insb. bis etwa pH 3 gesenkt werden.

Ebenso kann die Acetatstartkonzentration gemäß a) und b), für die vorstehend im wesentlichen ein Bereich von 0,5 - 2 % genannt wird, auch jenseits dieser Grenzen etwa zwischen 0,3 und 6 gewählt werden, wenn entsprechend aktive homoacetogene Bakterien zur Verfügung stehen. In jedem Falle ist die erfindungsgemäße Verbundfermentation in mindestens zwei Fermentern vorteilhaft.

Kernforschungsanlage Jülich GmbH    Pfeifer & Langen

## P a t e n t a n s p r ü c h e

1. Verfahren zur anaeroben Gewinnung von Essigsäure, ausgehend von Substraten für homoacetogene Bakterien, insbesondere von Zuckerlösungen, durch Gärung mit Hilfe von homoacetogenen Bakterien in wäßrigem Milieu, d a d u r c h g e k e n n z e i c h n e t, daß man die Fermentation in einem Fermenterverbund von zumindest zwei Fermentern durchführt.

2. Verfahren nach Anspruch 1, d a d u r c h g e k e n n z e i c h n e t, daß man die Fermentation in zwei im Wechselbetrieb arbeitenden Fermentern nach einem fed-batch-Verfahren durchführt, wobei jeweils ein solcher Teil des einen Fermenters vor Erreichen der Essigsäure-Endkonzentration in diesem Fermenter als Starter-Kultur und Essigsäure-Vorgabe für den anderen Fermenter verwendet wird, daß dessen Essigsäure- bzw. Acetatstart-Konzentration zwischen 0,5 und 2 %, insb. um ca. 1 % liegt.

3. Verfahren nach Anspruch 1, d a d u r c h g e k e n n z e i c h n e t, daß die Fermentation kontinuierlich in zwei aufeinanderfolgenden Fermentern durchgeführt wird, wobei die Verweilzeit im zweiten Fermenter zumindest gleich derjenigen im ersten ist.

T 1.814
ö/Fr

4. Verfahren nach Anspruch 3,
d a d u r c h g e k e n n z e i c h n e t , daß die Verweilzeit im zweiten Fermenter verdoppelt, insb. verdreifacht ist.

5. Verfahren nach einem der vorangehenden Ansprüche,
d a d u r c h g e k e n n z e i c h n e t , daß die Fermentation mit Hilfe von Acetogenium kivui durchgeführt wird.

6. Verfahren nach Anspruch 5,
d a d u r c h g e k e n n z e i c h n e t , daß die Nährlösung einen relativ hohen Spurenelementgehalt und einen relativ niedrigen Phosphatgehalt aufweist.

7. Verfahren nach Anspruch 5,
d a d u r c h g e k e n n z e i c h n e t , daß in zwei in Wechselbetrieb funktionierenden Fermentern (A und B) gearbeitet wird, indem jeweils aus einem Fermenter (A) ca. 36 - 42 Stunden nach Füllung bzw. Inbetriebnahme etwa 25 % seines Inhalts in den anderen Fermenter (B) überführt werden, der zu 75 % mit Nährlösung gefüllt ist, wonach der eine Fermenter (A) nach Erreichen der Endkonzentration (ca. 50 - 60 Stunden nach Füllung bzw. Inbetriebnahme) geleert und erneut zu 75 % mit frischer Nährlösung gefüllt wird.

8. Verfahren nach Anspruch 7, dadurch gekennzeichnet, daß die Gesamtmenge der umzusetzenden Glucose schrittweise zugegeben wird, wobei insb. jeweils mit Glucosenachlieferung zum Fermenter unter Aufrechterhaltung von etwa 2 % Glucose gearbeitet wird, bis insgesamt ca. 40 g/l Glucose zugegeben sind.

9. Verfahren nach Anspruch 7 oder 8, dadurch gekennzeichnet, daß man als erstes Inoculum für den Start der Verbundfermentation unter Vorgabe von 0,5 bis 2 % Acetat enthaltender Nährlösung eine Fermentation durchführt bis zur Erzielung einer Gesamt-Essigsäure-Konzentration > 4 %.

10. Verfahren nach Anspruch 9, dadurch gekennzeichnet, daß man als Bakterien Acetogenium kivui, einen pH von 6,2 bis 6,8 und eine Temperatur von 60 bis 70°C vorsieht und zunächst eine Fermentation, ausgehend von glucosehaltiger 0,5 bis 2%iger Acetatlösung durchführt, deren Glucosekonzentration durch wiederkehrende Nachlieferung von Glucose unter 4 %, insbesondere um 2 % gehalten wird, bis ca. 40 g/l Glucose zugegeben sind und eine Gesamtkonzentration von > 4 % Essigsäure erreicht ist.

11. Verfahren nach Anspruch 10, d a d u r c h g e k e n n z e i c h n e t , daß in der Startphase mit ca. 170 mM Acetatlösung bei pH 6,4 und 66°C gearbeitet wird.

12. Verfahren nach Anspruch 11, d a d u r c h g e k e n n z e i c h n e t , daß als 170 mM Acetatlösung für die erste Fermentation Natriumacetatlösung und zur Neutralisation jeweils Natronlauge (ca. 10 M) verwendet wird.

13. Verfahren nach Anspruch 5, d a d u r c h g e k e n n z e i c h n e t , daß bei einer kontinuierlichen Fermentation im ersten Fermenter mit einer Verweilzeit gearbeitet wird, die zur Bildung einer Essigsäurekonzentration von etwa 3 % ausreicht und im zweiten Fermenter eine etwa dreifache Verweilzeit eingestellt wird.

14. Verfahren nach Anspruch 13, d a d u r c h g e k e n n z e i c h n e t , daß im ersten Fermenter mit einer Verweilzeit von 18 - 24 Stunden und im zweiten Fermenter mit einer Verweilzeit von 54 - 75 Stunden gearbeitet wird.

15. Verfahren nach einem der vorangehenden Ansprüche, d a d u r c h g e k e n n z e i c h n e t , daß mit Immobilisierung der Biomasse im Fermenter oder ihrer Abtrennung vom Ablauf des Fermenters und Rückführung der abgetrennten Biomasse in den Fermenter gearbeitet wird.